# EUROPEAN PATENT SPECIFICATION

(11) **EP 0 415 924 B1**
(45) Date of publication and mention of the grant of the patent: **29.05.1996**
(21) Application number: 88909945.3
(22) Date of filing: 17.10.1988
(51) Int. Cl.: C12N 5/00, A61K 38/00

(54) **REGULATION OF CELL PROLIFERATION AND DIFFERENTIATION USING PEPTIDES**
REGULIERUNG DER ZELLENPROLIFERATION UND DIFFERENZIERUNG MITTELS PEPTIDEN
REGULATION DE LA PROLIFERATION ET DE LA DIFFERENCIATION CELLULAIRES PAR UTILISATION DE PEPTIDES

(30) Priority: 20.10.1987 US 110973
(43) Date of publication of application: 13.03.1991
(73) Proprietor: HOLICK, Michael F, Sudburry, MA 01776 (US)
(72) Inventor: HOLICK, Michael F, Sudburry, MA 01776 (US)
(74) Representative: Sheard, Andrew Gregory
(86) International application number: US8803649
(87) International publication number: WO8903873

(56) References cited:
- EP-A- 0 293 160
- US-A- 1 984 260
- BIOLOGICAL ABSTRACTS, vol. 81, no. 2, 15 January 1986, Philadelphia, PA (US); PARTRIDGE et al., AN 15489
- ENDOCRINOLOGY, vol. 118, no. 6, 1986, Baltimore, MD (US); MacDONALD et al., pp. 2445-2449
- JOURNAL OF INVESTIGATIVE DERMATOLOGY, vol. 86, no. 6, 01 June 1986, Baltimore, MD (US); SMITH et al., pp. 709-714
- SCIENCE, vol. 231, 24 January 1986, Washington, DC (US); MERENDINO et al., pp. 388-396
- BIOLOGICAL ABSTRACTS, vol. 82, no. 101, 15 November 1986, Philadelphia, PA (US); BIKLE et al., AN 93161
- JOURNAL OF CLINICAL ENDOCRINOLOGY & METABOLISM, vol. 65, no. 1,1987, Baltimore, MD (US); WU et a., pp. 105-109
- VERTEBRATE ENDOCRINOLOGY, Philadephia, PA (US), 1985; NORRIS, pp. 315-335
- SCIENCE, vol. 238, 1987; KEMP et al., pp. 1568-1570
- SCIENCE, vol. 237, 1987; SUVA et al., pp. 893-896
- ENDOCRINOLOGY, vol. 2, 1989; ROSENBLATT et al., pp. 848-891

## Description

### Background of the Invention

This invention relates to the regulation of cell differentiation and proliferation, e.g., for treatment of hyperproliferative skin disorders such as psoriasis.

Psoriasis is a disease of the epidermis and a major cause of disability and disfigurement for between 1 to 3% of the population of the world. In the United States approximately 2,000,000 to 8,000,000 persons suffer from this disorder, and approximately 100,000 are severly affected.

The disease is diagnosed by the presence of scaling, erythematous lesions on the scalp and extensor aspects of the arms and legs. Psoriatic lesions often are accentuated at sites of repeated trauma such as the elbows and knees. Furthermore, this skin disorder can afflict most of the areas of the skin of some individuals and can also cause internal damage such as arthritis. This disease is characterized by a hyperproliferation of the basal cells (a several fold increase in the number of basal cells of the epidermis). This increase in the basal cell population reduces the turnover time of the epidermis from the normal 27 days to 3-4 days. This shortened interval prevents normal cell maturation and keratinization, and this failure of maturation is reflected in an array of abnormal morphologic and biochemical changes. Numerous cytologic, histologic, histochemical, and biochemical alterations are known to be the result, rather than the cause, of the disease process.

The treatment of psoriasis still remains the province of dermatologists. The most effective treatment in the control of localized psoriasis for most patients is the topical use of corticosteroids with a plastic wrap, ultraviolet light treatments, and sunlight exposure. For certain patients with generalized psoriasis, it has been necessary to use a variety of systemic chemotherapeutic agents, especially methotrexate; the latter has the capacity to inhibit cell replication without a proportionate inhibition of cell function. Photochemotherapy was introduced in 1974, the so-called PUVA treatment. This treatment consists of administering psoralen prior to partial or whole body irradiation with a special light system that emits predominately long wave length ultraviolet light (UV-A).

Recently, it has been appreciated that the skin is not only the site for the synthesis of vitamin D, but is also a target tissue for its biologically active form, 1,25-dihydroxyvitamin D₃ (1,25(OH)₂D₃). A variety of tumor cells for example, HL-60, U937, M-1, as well as normal cells such as activated T-lymphocytes, monocytes, and cells isolated from the skin of rats, mice, humans and from cultured skin fibroblasts and keratinocytes contain high affinity (1.0 x 10⁻¹⁰M), low capacity, receptor-like protein for 1,25(OH)₂D₃. For tumor cells that possess a receptor for 1,25(OH)₂D₃, the hormone inhibits their proliferation and induces them to differentiate (Suda et al. in Vitamin D: Chemical, Biochemical and Clinical Update (Norman et al., ed.) Walter de Gruyter N.Y. 1985 pp. 187-196). 1,25(OH)₂D₃ inhibits the proliferation of human cultured fibroblasts and keratinocytes and induces keratinocytes to terminally differentiate (Smith et al. J. Invest. Dermatol. 86:709-714, 1986). It has been demonstrated that 1,25(OH)₂D₃ and its analog 1,24-dihydroxyvitamin D₃ are of value in the treatment of psoriasis when provided either orally or topically (Morimoto et al. Brit. J. Dermatol. 115:421-429, 1986; Kato et al. Brit. J. Dermatol. 115, 431-433, 1986).

Aging is associated with a variety of changes in the skin. There is an age-related decrease in epidermal turnover rate of approximately 30 to 50% between the 3rd and 8th decades. Thymidine labeling index (a measure of the proliferative activity of cells) of the epidermis in vivo has been reported to decline nearly 50% with age from approximately 5.1% in 19-25 year old men to approximately 2.85% in 69-85 year old men. Additional investigations have revealed that there is a 100% prolongation in stratum corneum replacement rate in old verses young men (Gilchrest, B. in Skin and Aging Processes, CRC Press, p. 21, 1984).

Parathyroid hormone (PTH), a polypeptide 84 amino acids long, plays an important role in the maintenance of the concentration of ionized calcium in extracellular fluids within normal range. Parathyroid hormone acts to raise extracellular calcium by its effects on the bone, kidney, and indirectly by increasing the production of 1,25(OH)₂D₃ to enhance intestinal calcium absorption (Krane S. M. and Potts, J. T. in Harrison's Textbook Principles of Internal Medicine, 9th ed., pp. 1824-1832, 1980). A variety of cells, including kidney cells, lymphocytes, and osteosarcoma cells, possess receptors for parathyroid hormone (Yamamoto, I. et al. J. Clin. Invest. 71:404-407, 1983; Goldring, S. et al. J. Clin. Endo. and Met. 46:425-433, 1978). A variety of in vitro and in vivo tests have been developed to assay for parathyroid hormone. These include the measurement of cAMP production in isolated canine kidney membranes (Nissenson et al. J. Clin. Endo. Metab. 52:840, 1981) and in osteosarcoma cells (Lindall, A.W. et al. J. Clin. Endo. Metab. 57:1007, 1983) and human fibroblasts (Goldring et al. J. Clin. Endocrinol. and Met. 46:425-433, 1978). A cytochemical assay has also been developed to measure glucose-6-phosphate dehydrogenase activity in guinea pig kidney (Goltzman, D. J. et al. Clin. Invest. 65:1309, 1980). In addition, a multiresponse parathyroid hormone assay has been developed to measure both agonist and antagonist properties of parathyroid hormone analogs (Horiuchi, N. et al. Am. J. Physiol. 244:E589-595, 1983).

A variety of analogs have been made of PTH. Of interest has been the observation that the 1-34 amino terminal fragment of the PTH molecule is biologically active in vitro and in vivo (Nussbaum, S. R. et al. J. Prot. Chem. 4:391-406, 1985). Rat PTH (1-34), which has a 5 amino acid sequence difference from the corresponding 1-34 region of both human and bovine PTH, has been found to be 8-10 fold more active than human PTH (1-34) and 2-4 fold more active than bovine PTH (1-34) in the canine adenylate cyclase system (Keutmann, H.T. et al. Endocrinol. 117, 1230, 1985). Structure activity studies revealed that PTH-stimulated biologic responses in vitro can be inhibited by [Nle⁸, Nle¹⁸, Tyr³⁴] bovine (3-34) PTH amide (bPTH-(3-34)). The parathyroid hormone analog [Tyr³⁴] bovine PTH-(7-34)-amide can inhibit the PTH-mediated elevation of plasma calcium in thyroparathyroidectomized rats in vivo, and is devoid of PTH-like agonist activity (Doppelt, S.H. et al. Proc. Natl. Acad. Sci. USA. 83;7557, 1986).

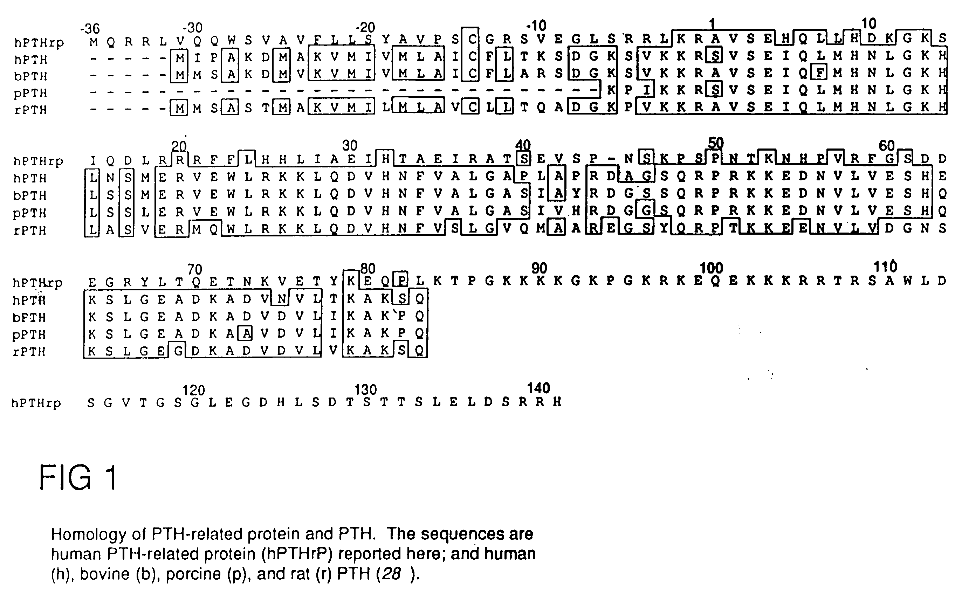

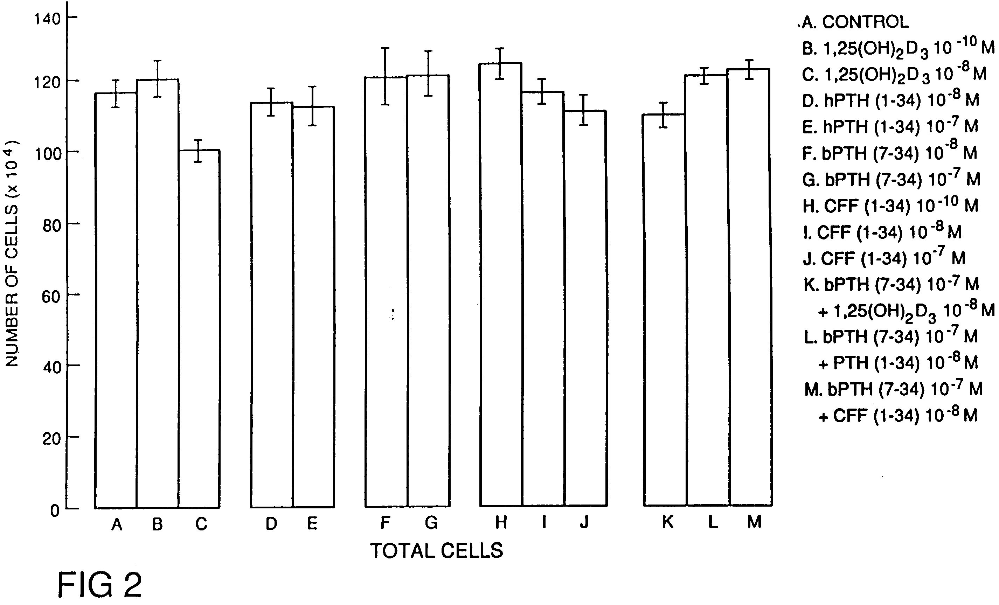

Recently, it has been found that cultured human keratinocytes make a parathyroid hormone-like protein, referred to herein as "hypercalcemic factor" (Merendino, J. J. et al. Science 231:388-390 1986). Keratinocyte-conditioned medium was harvested from confluent, first passage cultures. Conditioned medium from each of 10 keratinocyte cultures stimulated adenylate cyclase activity in a clonal ROS 17-2.8 cell assay. This biologically active substance is thought to be one of the factors responsible for causing humoral hypercalcemia in patients with a variety of malignancies. This PTH-related protein was isolated from a human lung cancer cell line, and full-length complementary DNA clones encoding it have been inserted into expression vectors used to produce the peptide in mammalian cells. The clones were found to encode a prepropeptide of 36 amino acids and a mature protein of 141 amino acids that has significant homology with parathyroid hormone in the amino terminal region; of the first 16 residues of this protein, 8 of the 16 were found to be identical to human PTH (Suva, L.J. et al. Science 232;893-896, 1987). Fig. 1 herein, reproduced from Fig. 2 of Suva et al., gives the sequences of the two proteins.

### Summary of the Invention

The aforementioned seemingly divergent lines of research, i.e., the use of 1,25(OH)₂D₃ for the treatment of psoriasis based on its ability to inhibit keratinocyte proliferation and induce terminal differentiation, and the observation that cultured human keratinocytes produce the PTH-like-hypercalcemic factor have been brought together in the present invention. This invention arose in part out of the following discoveries: 1) when human cultured keratinocytes are incubated with human PTH (1-34) (herein, PTH (1-34)) at 10⁻⁷ and 10⁻⁸M, this polypeptide fragment inhibits growth and induces terminal differentiation of these cells; 2) a synthetic fragment of hypercalcemic factor ([Tyr³⁴] calcemic factor fragment (1-34) amide (herein, CFF (1-34)) also inhibits the proliferation and induces terminal differentiation of keratinocytes in a dose-dependent manner at 10⁻¹⁰, 10⁻⁸ and 10⁻⁷M concentrations; 3) [Nle⁸, Nle¹⁸, Tyr³⁴] bovine (3-34) PTH amide (herein, PTH (3-34)), like PTH (1-34), is an agonist, i.e., it inhibits proliferation and induces terminal differentiation of keratinocytes; 4) [Tyr³⁴] bovine PTH (7-34) amide (herein, PTH (7-34); is an antagonist which blocks the antiproliferative and differentiation inducing action of PTH (1-34) and CFF (1-34); and 5) the antagonist PTH (7-34) blocks the keratinocyte proliferation and terminal differentiation inducing activity of 1,25(OH)₂D₃.

Thus the invention generally provides two important, but opposite, therapeutic methods, one involving proliferation inhibition and differentiation enhancement, the other involving proliferation enhancement.

Thus in a first aspect the invention relates to the use of a peptide having 10% or greater homology with the amino terminal 34 amino acid region of human parathyroid hormone (PTH) or with the amino terminal 34 amino acid region of human hypercalcemic factor, the peptide being capable of enhancing the differentiation and inhibiting the proliferation in vitro of cultured human keratinocytes, in the preparation of an agent for inhibiting proliferation and enhancing differentiation of a mammalian cell.

This first aspect of the invention thus involves inhibiting proliferation and enhancing differentiation of a mammalian cell by bringing the cell into contact with a peptide (preferably at least 3, and more preferably at least 8, amino acids long) which has 10% or greater (more preferably, 50% or greater, and most preferably 75% or greater) homology with the amino-terminal 34 amino acid region of human parathyroid hormone or human hypercalcemic factor, and which is capable of inhibiting proliferation or enhancing the differentiation in vitro of cultured human keratinocytes.

A second aspect of the invention relates to the use of a peptide having 10% or greater homology with the amino terminal 34 amino acid region of human parathyroid hormone (PTH) or with the amino terminal 34 amino acid region of human hypercalcemic factor, the peptide being capable of blocking the inhibition of proliferation of cultured human keratinocytes by PTH(1-34), 1,25(OH)₂D₃ or CFF(1-34) or of blocking the stimulation of differentiation in vitro of cultured human keratinocytes by PTH(1-34), 1,25(OH)₂D₃, or CFF(1-34), in the preparation of an agent for inducing proliferation of a mammalian cell.

This second aspect of the invention thus involves enhancing proliferation of a mammalian cell by bringing the cell into contact with a peptide (preferably at least 3, and more preferably at least 8, amino acids long) which has 10% or greater (more preferably, 50% or greater, and most preferably 75% or greater) homology with the amino-terminal 34 amino acid region of human parathyroid hormone or human hypercalcemic factor, and which is capable of blocking the differentiation or the inhibition of proliferation in vitro of cultured human keratinocytes, by PTH (1-34) or 1,25(OH)₂D₃ or CFF (1-34).

The first aspect, inducing differentiation of cells while inhibiting their proliferation, has particular application in the treatment of hyperproliferative skin disorders such as psoriasis. The method may also be useful in the treatment of certain cancers, by the inhibition of cancer cell proliferation and by the induction of differentiation.

The second aspect, blocking antiproliferative activity of PTH (1-34), CFF (1-34) or 1,25(OH)₂D₃, can have applications in the promotion of skin growth in patients with burns, skin ulcerations, and wounds, as well as in the stimulation of epidermal regrowth in people who have decreased proliferative activity due to aging.

A third aspect of the invention relates to the use of, PTH(1-34), CFF(1-34), PTH(3-34), PTH(1-38), PTH(1-44), human hypercalcemic factor (1-141) or human hypercalcemic factor (1-40) in the preparation of an agent for the treatment of a hyperproliferative disease such as psoriasis or cancer.

A fourth aspect encompasses the use of PTH(7-34) in the preparation of an agent for treating burns, ulcerations or wounds, for stimulating epidermal regrowth in people who have decreased proliferative activity due to aging, or for stimulating hair growth.

Other features and advantages of the invention will be apparent from the following description of the preferred embodiments thereof, and from the claims.

### Description of the Preferred Embodiments

The drawings are first described.

### Drawings

Figure 1 is a comparison of the amino acid sequences of human PTH and hypercalcemic factor, taken from Suva et al., id.

Fig. 2, 3, 4, 5 are bar graphs showing comparative proliferative effects of peptides of the invention.

Figs. 6 and 7 are graphs showing cell differentiation effects of peptides of the invention.

### Synthesis and Selection of Peptides

The peptides used in the methods of the invention are all easily synthesized, using recombinant DNA or solid phase peptide synthesis techniques, and some are available commercially as well, or can be derived from commercially available peptides. For example, there is reproduced below a section of the Bach Chem catalog, listing a number of available human, rat, and bovine analogs and fragments. (The Peninsula Laboratory catalog also lists available fragments.)

Hypercalcemia of Malignancy Factor (1-40)
(PTH-like Adenylate Cyclase-Stimulating Protein)
(ACSP)
H2N-Ala-Val-Ser-Glu-His-Gln-Leu-Leu-His-Asp-Lys-Gly-Lys-Ser-Ile-
Gln-Asp-Leu-Arg-Arg-Arg-Phe-Phe-Leu-His-His-Leu-Ile-Ala-Glu-Ile-
His-Thr-Ala-Glu-Ile-Arg-Ala-Thr-Ser-OH

Parathyroid Hormone, Bovine (84 amino acids)
H2N-Ala-Val-Ser-Glu-Ile-Gln-Phe-Met-His-Asn-Leu-Gly-Lys-His-Leu-
Ser-Ser-Met-Glu-Arg-Val-Glu-Trp-Leu-Arg-Lys-Lys-Leu-Gln-Asp-Val-
His-Asn-Phe-Val-Ala-Leu-Gly-Ala-Ser-Ile-Ala-Try-Arg-Asp-Gly-Ser-
Ser-Gln-Arg-Pro-Arg-Lys-Lys-Glu-Asp-Asn-Val-Leu-Val-Glu-Ser-His-
Gln-Lys-Ser-Leu-Gly-Glu-Ala-Asp-Lys-Ala-Asp-Val-Asp-Val-Leu-Ile-
Lys-Ala-Lys-Pro-Gln-OH

[Tyr63]-Parathyroid Hormone (63-84), Human
H2N-Tyr-Glu-Lys-Ser-Leu-Gly-Glu-Ala-Asp-Lys-Ala-Asp-Val-Asn-Val-
Leu-Thr-Lys-Ala-Lys-Ser-Gln-OH

Parathyroid Hormone (64-84), Human
H2N-Glu-Lys-Ser-Leu-Gly-Glu-Ala-Asp-Lys-Ala-Asp-Val-Asn-Val-Leu-
Thr-Lys-Ala-Lys-Ser-Gln-OH

[Try69]-Parathyroid Hormone (69-84), Human
H2N-Tyr-Ala-Asp-Lys-Ala-Asp-Val-Asn-Val-Leu-Thr-Lys-Ala-Lys-Ser-
Gln-OH

Parathyroid Hormone (70-84), Human
H2N-Ala-Asp-Lys-Ala-Asp-Val-Asn-Val-Leu-Thr-Lys-Ala-Lys-Ser-Gln-
OH

Human Bone Gla Protein (37-49)(BGP 37-49)
H2N-Gly-Phe-Gln-Glu-Ala-Tyr-Arg-Arg-Phe-Tyr-Gly-Pro-Val-OH
Ref: Poser, J.W. et al. (1980) PNAS 255:8685

[Tyr38, Phe42,46]-Human Bone Gla Protein (38-49)
H2N-Tyr-Gln-Glu-Ala-Phe-Arg-Arg-Phe-Phe-Gly-Pro-Val-OH

Human Bone Gla Protein (45-49)
H2N-Phe-Tyr-Gly-Pro-Val-OH

Parathyroid Hormone, Human (84 amino acids)
H2N-Ser-Val-Ser-Glu-Ile-Gln-Leu-Met-His-Asn-Leu-Gly-Lys-His-Leu-
Asn-Ser-Met-Glu-Arg-Val-Glu-Trp-Leu-Arg-Lys-Lys-Leu-Gln-Asp-Val-
His-Asn-Phe-Val-Ala-Leu-Gly-Ala-Pro-Leu-Ala-Pro-Arg-Asp-Ala-Gly-
Ser-Gln-Arg-Pro-Arg-Lys-Lys-Glu-Asp-Asn-Val-Leu-Val-Glu-Ser-His-
Glu-Lys-Ser-Leu-Gly-Glu-Ala-Asp-Lys-Ala-Asp-Val-Asn-Val-Leu-Thr-
Lys-Ala-Lys-Ser-Gln-OH
Ref: Kimura, T. et al (1983) BBRC 114:493; Fairwell, T. et
al., (1983) Biochemistry 22:2691.

Parathyroid Hormone, Rat (84 amino acids)
H2N-Ala-Val-Ser-Glu-Ile-Gln-Leu-Met-His-Asn-Leu-Gly-Lys-His-Leu-
Ala-Ser-Val-Glu-Arg-Met-Gln-Trp-Leu-Arg-Lys-Lys-Leu-Gln-Asp-Val-
His-Asn-Phe-Val-Ser-Leu-Gly-Val-Gln-Met-Ala-Ala-Arg-Glu-Gly-Ser-
Tyr-Gln-Arg-Pro-Thr-Lys-Lys-Glu-Asp-Asn-Val-Leu-Val-Asp-Gly-Asn-
Ser-Lys-Ser-Leu-Gly-Glu-Gly-Asp-Lys-Ala-Asp-Val-Asp-Val-Leu-Val-
Lys-Ala-Lys-Ser-Gln-OH
Ref: Heinrich, G. et al., (1984) J. Biol. Chem. 259:3320

Parathyroid Hormone (1-34), Bovine
H2N-Ala-Val-Ser-Glu-Ile-Gln-Phe-Met-His-Asn-Leu-Gly-Lys-His-Leu-
Ser-Ser-Met-Glu-Arg-Val-Glu-Trp-Leu-Arg-Lys-Lys-Leu-Gln-Asp-Val-
His-Asn-Phe-OH
Ref: Tregear, G.W. et al (1977) Biochemistry 16:2817

Parathyroid Hormone (1-34), Human
H2N-Ser-Val-Ser-Glu-Ile-Gln-Leu-Met-His-Asn-Leu-Gly-Lys-His-Leu-
Asn-Ser-Met-Glu-Arg-Val-Glu-Trp-Leu-Arg-Lys-Lys-Leu-Gln-Asp-Val-
His-Asn-Phe-OH
Ref: Takai, T. et al. (1979) Peptide Chemistry

Parathyroid Hormone (1-34) Biotinyl, Human
Biotin-Ser-Val-Ser-Glu-Ile-Gln-Leu-Met-His-Asn-Leu-Gly-Lys-His-
Leu-Asn-Ser-Met-Glu-Arg-Val-Glu-Trp-Leu-Arg-Lys-Lys-Leu-Gln-Asp-
Val-His-Asn-Phe-OH

Parathyroid Hormone (1-34), Rat
H2N-Ala-Val-Ser-Glu-Ile-Gln-Leu-Met-His-Asn-Leu-Gly-Lys-His-Leu-
Ala-Ser-Val-Glu-Arg-Met-Gln-Trp-Leu-Arg-Lys-Lys-Leu-Gln-Asp-Val-
His-Asn-Phe-OH
Ref: Kaufmann H.T. et al. (1984) abstract, ASBMR Conf.

[Nle8,18, Tyr34]-Parathyroid Hormone (1-34), Amide, Bovine
H2N-Ala-Val-Ser-Glu-Ile-Gln-Phe-Nle-His-Asn-Leu-Gly-Lys-His-Leu-
Ser-Ser-Nle-Glu-Arg-Val-Glu-Trp-Leu-Arg-Lys-Lys-Leu-Gln-Asp-Val-
His-Asn-Tyr-NH2
Ref: Coltrera, M.D. et al. (1981) J. Biol. Chem. 256:10,555;
Rosenblatt, M. et al. (1977) Endocr. Res. Comm 4:115;
Rosenblatt, M. et al. (1976) J. Biol. Chem. 251:159.

[Nle8,18, Tyr34]-Parathyroid Hormone (1-34), Human
H2N-Ser-Val-Ser-Glu-Ile-Gln-Leu-Nle-His-Asn-Leu-Gly-Lys-His-Leu-
Asn-Ser-Nle-Glu-Arg-Val-Glu-Trp-Leu-Arg-Lys-Lys-Leu-Gln-Asp-Val-
His-Asn-Tyr-OH

[Nle8,21, Tyr34]-Parathyroid Hormone (1-34), Amide, Rat
H2N-Ala-Val-Ser-Glu-Ile-Gln-Leu-Nle-His-Asn-Leu-Gly-Lys-His-Leu-
Ala-Ser-Val-Glu-Arg-Nle-Gln-Trp-Leu-Arg-Lys-Lys-Leu-Gln-Asp-Val-
His-Asn-Tyr-NH2

[Tyr1]-Parathyroid Hormone (1-34), Human
H2N-Tyr-Val-Ser-Glu-Ile-Gln-Leu-Met-His-Asn-Leu-Gly-Lys-His-Leu-
Asn-Ser-Met-Glu-Arg-Val-Glu-Trp-Leu-Arg-Lys-Lys-Leu-Gln-Asp-Val-
His-Asn-Phe-OH

Parathyroid Hormone (1-38), Human
H2N-Ser-Val-Ser-Glu-Ile-Gln-Leu-Met-His-Asn-Leu-Gly-Lys-His-Leu-
Asn-Ser-Met-Glu-Arg-Val-Glu-Trp-Leu-Arg-Lys-Lys-Leu-Gln-Asp-Val-
His-Asn-Phe-Val-Ala-Leu-Gly-OH
Ref: Hosch, R.D. et al. (1984) Horm. Metab. Res. 16:559

Parathyroid Hormone (1-44), Human
H2N-Ser-Val-Ser-Glu-Ile-Gln-Leu-Met-His-Asn-Leu-Gly-Lys-His-Leu-
Asn-Ser-Met-Glu-Arg-Val-Glu-Trp-Leu-Arg-Lys-Lys-Leu-Gln-Asp-Val-
His-Asn-Phe-Val-Ala-Leu-Gly-Ala-Pro-Leu-Ala-Pro-Arg-OH
Ref: Kimura, T. et al. (1981) Biopolymers 20:1823

Parathyroid Hormone (3-34), Bovine
H2N-Ser-Glu-Ile-Gln-Phe-Met-His-Asn-Leu-Gly-Lys-His-Leu-Ser-Ser-
Met-Glu-Arg-Val-Glu-Trp-Leu-Arg-Lys-Lys-Leu-Gln-Asp-Val-His-Asn-
Phe-OH
Ref: Lowik, C. et al., (1985) Cell Calcium 6:311

[Nle8,18, Tyr34]-Parathyroid Hormone (3-34), Amide, Bovine
H2N-Ser-Glu-Ile-Gln-Phe-Nle-His-Asn-Leu-Gly-Lys-His-Leu-Ser-Ser-
Nle-Glu-Arg-Val-Glu-Trp-Leu-Arg-Lys-Lys-Leu-Gln-Asp-Val-His-Asn-
Tyr-NH2
Ref: Rosenblatt, M. et al. (1977) J. Biol. Chem 252:5847

[Nle8,18, Tyr34]-Parathyroid Hormone (7-34), Amide, Bovine
H2N-Phe-Nle-His-Asn-Leu-Gly-Lys-His-Leu-Ser-Ser-Nle-Glu-Arg-Val-
Glu-Trp-Leu-Arg-Lys-Lys-Leu-Gln-Asp-Val-His-Asn-Tyr-NH2

[Tyr34]-Parathyroid Hormone (7-34), Amide, Bovine
(Parathyroid Hormone Antagonist)
H2N-Phe-Met-His-Asn-Leu-Gly-Lys-His-Leu-Ser-Ser-Met-Glu-Arg-Val-
Glu-Trp-Leu-Arg-Lys-Lys-Leu-Gln-Asp-Val-His-Asn-Tyr-NH2

Parathyroid Hormone (13-34), Human
H2N-Lys-His-Leu-Asn-Ser-Met-Glu-Arg-Val-Glu-Trp-Leu-Arg-Lys-Lys-
Leu-Gln-Asp-Val-His-Asn-Phe-OH

[Tyr27]-Parathyroid Hormone (27-48), Human
H2N-Tyr-Leu-Gln-Asp-Val-His-Asn-Phe-Val-Ala-Leu-Gly-Ala-Pro-Leu-
Ala-Pro-Arg-Asp-Ala-Gly-Ser-OH

Parathyroid Hormone (28-48), Human
H2N-Leu-Gln-Asp-Val-His-Asn-Phe-Val-Ala-Leu-Gly-Ala-Pro-Leu-Ala-
Pro-Arg-Asp-Ala-Gly-Ser-OH
Ref: Rosenblatt, M. et al., (1977) Biochemistry 16:2811

[Tyr43]-Parathyroid Hormone (43-68), Human
H2N-Tyr-Arg-Asp-Ala-Gly-Ser-Gln-Arg-Pro-Arg-Lys-Lys-Glu-Asp-Asn-
Val-Leu-Val-Glu-Ser-His-Glu-Lys-Ser-Leu-Gly-OH

Parathyroid Hormone (44-68), Human
H2N-Arg-Asp-Ala-Gly-Ser-Gln-Arg-Pro-Arg-Lys-Lys-Glu-Asp-Asn-Val-
Leu-Val-Glu-Ser-His-Glu-Lys-Ser-Leu-Gly-OH
Ref: Rosenblatt, M., Et al., (1977) J. Med. Chem. 20:1452

[Tyr52]-Parathyroid Hormone (52-84), Human
H2N-Tyr-Lys-Lys-Glu-Asp-Asn-Val-Leu-Val-Glu-Ser-His-Glu-Lys-Ser-
Leu-Gly-Glu-Ala-Asp-Lys-Ala-Asp-Val-Asn-Val-Leu-Thr-Lys-Ala-Lys-
Ser-Gln-OH

Parathyroid Hormone (53-84), Human
H2N-Lys-Lys-Glu-Asp-Asn-Val-Leu-Val-Glu-Ser-His-Glu-Lys-Ser-Leu-
Gly-Glu-Ala-Asp-Lys-Ala-Asp-Val-Asn-Val-Leu-Thr-Lys-Ala-Lys-Ser-
Gln-OH
Ref: Rosenblatt, M., et al (1978) Endocrinology 103:978

When selecting a candidate peptide for either method of the invention, a preferred first step is to choose a peptide which includes a fragment which is at least 10%, and more preferably 50% or more, homologous with an 8 or greater amino acid long fragment within the amino terminal 34 amino acid region of human PTH or hypercalcemic factor. Because of the high degree of homology among human PTH and PTH of other species, non-human as well as human fragments or analogs can be used. Further, the fragment can be modified in any of a variety of standard chemical ways, e.g., the carboxy-terminal amino acid residue can be made into a terminal amide group; the amino-terminal residue can be modified with groups to, e.g., enhance lipophilicity; the peptide can be chemically glycosylated to increase solubility or in vivo half-life; and D-amino acids can be substituted for L-isomers in the peptide.

Where the peptide is to be used to inhibit proliferation, it is generally desirable to include some or all of the amino terminal six amino acids of PTH or hypercalcemic factor, while, generally, fewer or none of those amino acids are used where the peptide is to be used to inhibit antiproliferative effects and differentiation.

Candidate peptides are tested for suitability as inhibitors or enhancers of cell proliferation and differentiation using cultured human keratinocytes, as follows. Those peptides which inhibit proliferation and induce differentiation in cultured keratinocytes are those potentially useful as therapeutic agents in treating disorders, e.g., psoriasis and cancer, where suppression of cell prcliferation is desired, while those peptides which block the effect of those agonist peptides or 1,25(OH)₂D₃ are those potentially useful as therapeutic agents in treating disorders, e.g., burns, skin ulcerations, wounds where maintenance or stimulation of cell proliferation is desired.

The peptides which block antiproliferative compounds can also be useful in conjunction with chemotherapeutic agents in the treatment of cancer; many chemotherapeutic agents are effective only against dividing cells, and the blocking peptides can have the effect of inducing division of otherwise dormant cells, rendering them vulnerable to the chemotherapy. The blocking peptides can also be used to stimulate and maintain hair growth. The blocking peptides can also be useful in promoting growth of new cells, e.g., skin cells, in topical skin creams. The differentiation-inducing peptides can be used as immunostimulants, by inducing maturation of monocytes and lymphocytes bearing PTH receptors, while the blocking peptides can be used to inhibit lymphocyte maturation, and thus can be used to treat conditions, e.g., autoimmune diseases such as juvenile diabetes, rheumatoid arthritis, and allograft rejection, where mature lymphocytes are a causative agent.

### Keratinocyte Culture

Keratinocytes were grown in culture as follows. NIH 3T3 cells were plated at 0.5 x 10⁵ cells per 35-mm tissue-culture dish, and two days later were lethally irradiated with a cobalt-60 source (5000 rads). Keratinocytes were obtained from neonatal foreskin after overnight trypsinization at 4°C and treatment with 0.02% EDTA. Keratinocytes were plated in 2 ml of serum-free medium per dish on the lethally irradiated 3T3 cells. Each experiment was performed on primary or secondary keratinocyte cultures obtained from different skin samples. The serum-free medium consisted of Dulbecco's Modified Eagle's Medium (DMEM) with high (1.8 mM) concentration of calcium (M.A. Bioproducts, Walkersville, MD) containing 7 growth factors: epidermal growth factor (25 ng/ml); hydrocortisone (203 ng/ml); insulin (5 µg/ml); prostaglandin E₁ (50 ng/ml); transferrin (5 µg/ml; prostaglandin E₁ (50 ng/ml); cholera toxin (0.1 µg/ml); (Sigma Chemical Co., St. Louis, MO); and selenous acid (2 ng/ml) (Collaborative Research, Lexington, MA). At one week in culture, hydrocortisone and cholera toxin were removed from the medium, and the dishes were washed with 0.02% EDTA to remove any remaining 3T3 cells.

Four peptides were tested, at various concentrations, in keratinocyte culture: CFF (1-34); PTH (1-34); PTH (3-34); and PTH (7-34). In addition, 1,25-(OH)₂D₃ was tested, alone and in combination with PTH (7-34), and PTH (1-34) and PTH (7-34) were tested together.

### Quantitation of Morphological Changes During Keratinocyte Differentiation

Beginning at one week in culture, groups of triplicate plates of keratinocytes were incubated with the aforementioned compounds or vehicle alone. After one week of dosing, the medium was removed from each culture, spun down, and resuspended for the counting of the desquamated floater cells. A hemacytometer was used to count the different cell types under a phase-contrast microscope. The attached cells were then trypsinized for 30-40 min with 0.1 EDTA and 0.1% trypsin and then neutralized with medium. The keratinocytes were spun down and resuspended in a known volume of medium. Duplicate aliquots were taken for counting the basal (small, rounded) and squamous (larger, irregular-shaped, flattened) cells. The remaining cells were spun down and treated with 10 mM Tris-HCl (pH 7.4) with 1% beta-mercaptoethanol and 1% sodium dodecyl sulfate (SDS) at room temperature for 10 min. Only cells with cornified envelopes were present after this treatment.

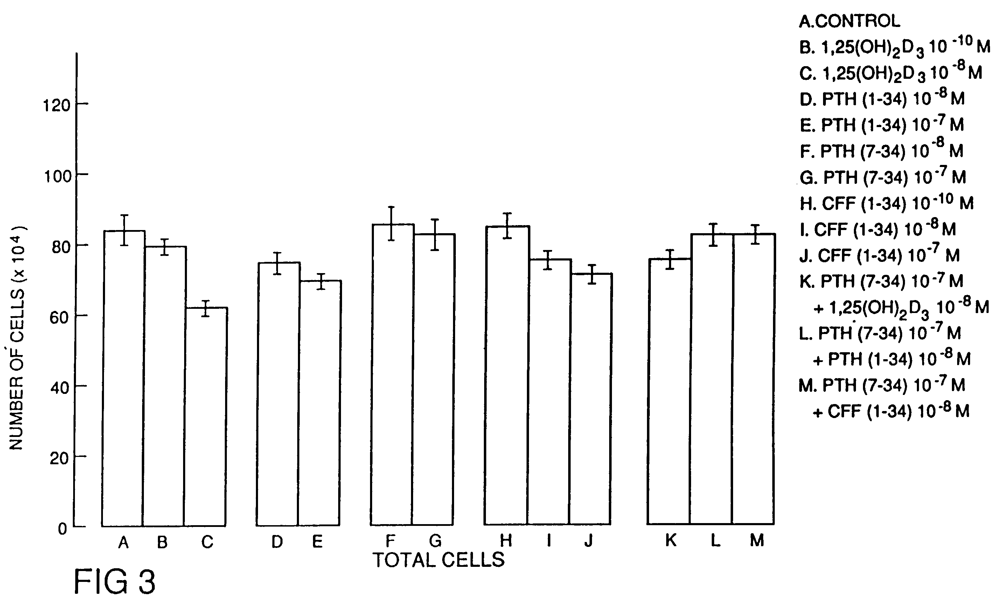

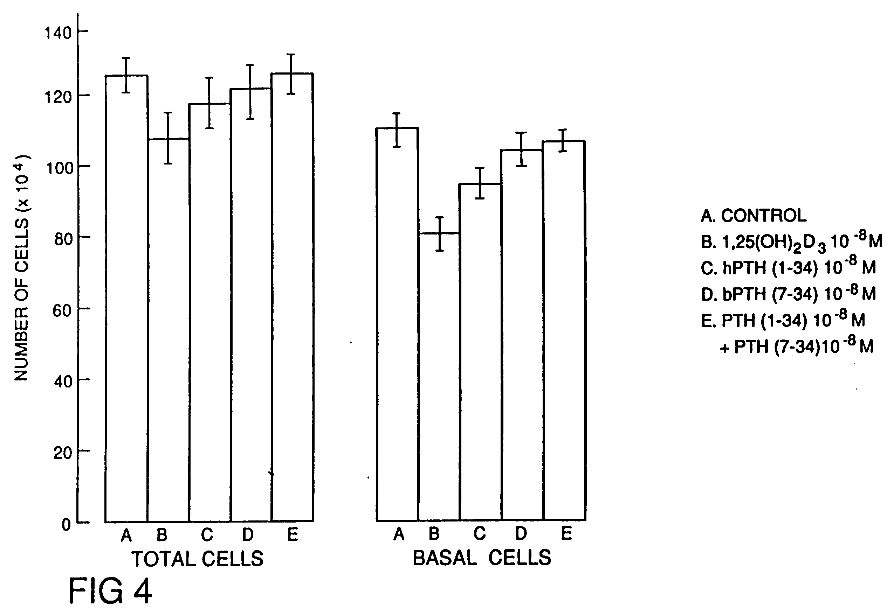

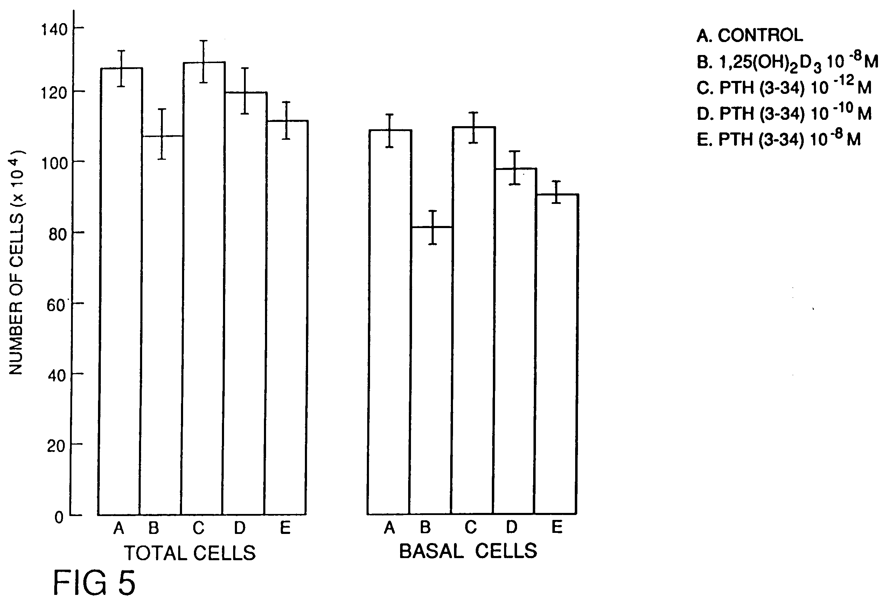

After seven days the cultures were evaluated for their total cell content, number of basal cells, and cornified envelopes as described above. As can be seen in Figures 2, 3, 4 and 5, 1,25(OH)₂D₃ in a dose-dependent manner decreased the total number of cells and the number of basal cells in the cultures. In a similar manner, PTH (1-34) at 10⁻⁸M and 10⁻⁷M, and PTH (3-34) at 10⁻¹⁰ and 10⁻⁸M significantly decreased the total number of basal cells, as did CFF (1-34) at 10⁻⁸M and 10⁻⁷M. PTH (7-34) at 10⁻⁸M and 10⁻⁷M had no effect. Furthermore, when 1,25(OH)₂D₃ at 10⁻⁸M was co-incubated with bPTH (7-34) at 10⁻⁷M, the PTH antagonist significantly decreased the response to 1,25(OH)₂D₃. Similarly, when PTH (7-34) was co-incubated with hPTH (1-34) or CFF (1-34), the PTH (7-34) inhibited the effect of PTH (1-34) and CFF (1-34) on the total number of cells and total number of basal cells in the cultures.

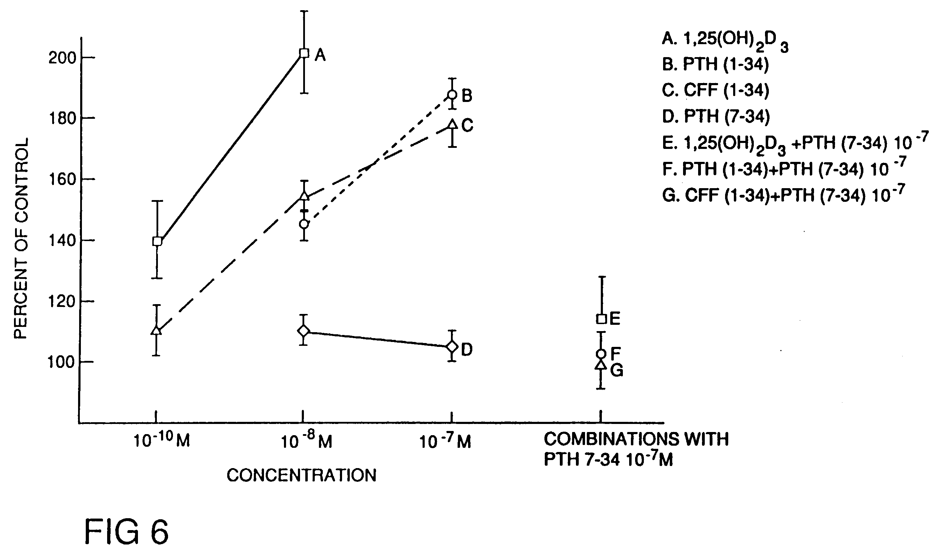

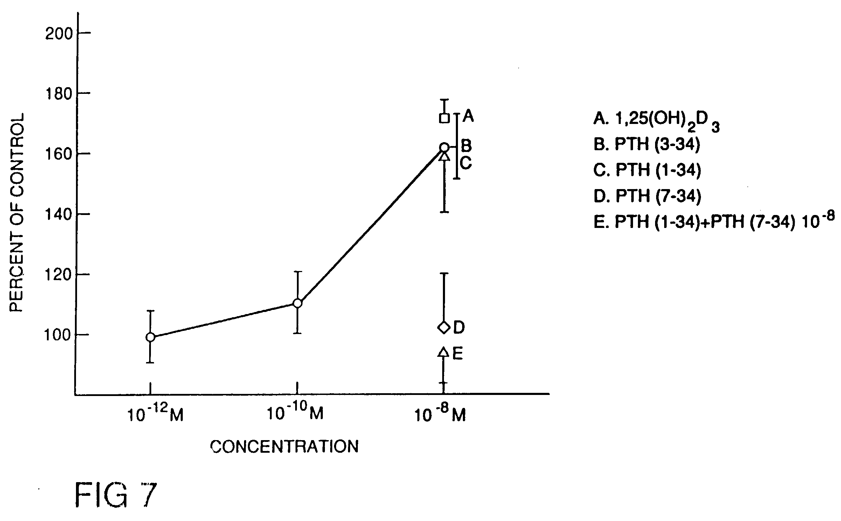

Figures 6 and 7 represent the percent of control of the total number of cornified envelopes observed in the cultures. As can be seen in Figure 6, 1,25(OH)₂D₃ increased, in a dose-dependent manner, the total number of cornified envelopes. Similarly, PTH (1-34), PTH (3-34), and CFF (1-34) also increased the number of cornified envelopes relative to the control cultures in a dose-dependent manner. PTH (7-34) at 10⁻⁸M and 10⁻⁷M had no effect. Furthermore, PTH (7-34) inhibited the effect of 1,25(OH)₂D₃, PTH (1-34), and CFF (1-34) on cornified envelope development.

These results demonstrate that 1) PTH (1-34) is biologically effective in inhibiting the proliferation of cultured human keratinocytes and inducing terminal differentiation, and is thus a candidate for use as a therapeutic for treatment of hyperproliferative diseases such as psoriasis and cancer; 2) CFF (1-34) and PTH (3-34) have the same effect as hPTH (1-34) and are thus also candidates for hyperproliferative disease therapy; 3) PTH (7-34), an antagonist to PTH action, has substantially no effect on proliferation or terminal differentiation by itself, but can block the activity of 1,25(OH)₂D₃, PTH (1-34), and CFF (1-34) in inhibiting proliferation or inducing termination differentiation of cultured human keratinocytes, and thus is a candidate for repair therapies, e.g., treating burns, ulcerations, and wounds.

### Use

The peptides are administered in therapeutically effective amounts to people in need of them. Generally, the peptides are administered admixed with a pharmaceutically acceptable carrier substance, e.g., magnesium carbonate or lactose. The composition can be in the form of a pill, tablet, capsule, liquid, or sustained release tablet for oral administration; a liquid for nasal administration; or a liquid for intravenous, subcutaneous, parenteral, or intraperitoneal administration.

Dosage will be dependent upon the age, health, and weight of the recipient, kind of concurrent treatment, if any, frequency of treatment, and the nature of the effect desired. Generally, daily dosage will be from about 0.001 micrograms/kg to 10,000 micrograms/kg, preferably 0.1 to 10 micrograms per kg of body weight. Normally, from 0.1 to 1000 micrograms/kg per day, in one or more applications per day, will be effective to obtain the desired results. The compounds, in addition to being capable of being administered as described above can be used in a pharmacologically inert topical carrier such as one comprising a gel, an ointment or a cream, including such carriers as water, glycerol, alcohol, propylene glycol, fatty alcohols, triglycerides, fatty acid esters or mineral oils. Other possible carriers are liquid petrolatum, isopropylpalmitate, polyethylene glycol ethanol 95%, polyoxyethylene monolaurate 5% in water, sodium lauryl sulfate 5% in water, and the like. Materials such as anti-oxidants, humectants, viscosity stabilizers and the like may be added, if necessary.

The compounds can also be administered by means of subcutaneous pumps, patches, tapes or by means of liposomal carriers. The peptides can be provided in the form of pharmaceutically acceptable salts. Examples of preferred salts are those of therapeutically acceptable organic acids, e.g., acetic, lactic, maleic, citric, malic, ascorbic, succinic, benzoic, salicylic, methanesulfonic, toluenesulfonic, or pamoic acid, as well as polymeric acids such as tannic acid or carboxymethyl cellulose, and salts with inorganic acids such as hydrohalic acids, e.g., hydrochloric acid, sulfuric acid, or phosphoric acid.

## Claims

1. The use of a peptide having 10% or greater homology with the amino terminal 34 amino acid region of human parathyroid hormone (PTH) or with the amino terminal 34 amino acid region of human hypercalcemic factor, the peptide being capable of enhancing the differentiation and inhibiting the proliferation *in vitro* of cultured human keratinocytes, in the preparation of an agent for inhibiting proliferation and enhancing differentiation of a mammalian cell.

2. The use according to claim 1 in the preparation of an agent for treating a hyperproliferative skin disease or for treating cancer.

3. The use according to claim 2 in the preparation of an agent for treating psoriasis.

4. The use as claimed in any preceding claim wherein the peptide has greater than 50% homology with said amino terminal region.

5. The use as claimed in any preceding claim wherein the peptide is PTH(1-34), CFF(1-34), PTH(1-84), PTH(3-34), PTH(1-38), PTH(1-44), human hypercalcemic factor (1-141), or human hypercalcemia of malignancy factor (1-40).

6. The use of a peptide having 10% or greater homology with the amino terminal 34 amino acid region of human parathyroid hormone (PTH) or with the amino terminal 34 amino acid region of human hypercalcemic factor, the peptide being capable of blocking the inhibition of proliferation of cultured human keratinocytes by PTH(1-34), 1,25(OH)₂D₃ or CFF(1-34) or of blocking the stimulation of differentiation *in vitro* of cultured human keratinocytes by PTH(1-34), 1,25(OH)₂D₃, or CFF(1-34), in the preparation of an agent for inducing proliferation of a mammalian cell.

7. The use according to claim 6 in the preparation of an agent for the promotion of skin growth in a patient with burns, skin ulcerations, or wounds, for stimulating epidermal regrowth in a patient with decreased proliferative activity due to aging, or for stimulating hair growth.

8. The use as claimed in claim 6 or claim 7 wherein the peptide has a greater than 50% homology with said amino terminal region.

9. The use as claimed in any of claims 6 to 8 wherein the peptide is PTH(7-34).

## Patentansprüche

1. Verwendung eines Peptids mit 10 % oder mehr Homologie zur Region der 34 amino-terminalen Aminosäuren des humanen Parathyroidhormons (PTH) oder der Region der 34 amino-terminalen Aminosäuren des humanen Hypercalcämiefaktors, wobei das Peptid die Differenzierung von kultivierten humanen Keratinocyten verstärken oder deren Vermehrung in vitro inhibieren kann, zur Herstellung eines Mittels zur Inhibierung der Vermehrung und zur Verstärkung der Differenzierung von Säugetierzellen.

2. Verwendung nach Anspruch 1 zur Herstellung eines Mittels zur Behandlung einer hyperproliferativen Hautkrankheit oder zur Behandlung von Krebs.

3. Verwendung nach Anspruch 2 zur Herstellung eines Mittels zur Behandlung von Psoriasis.

4. Verwendung nach einem der vorstehenden Ansprüche, worin das Peptid zu mehr als 50 % homolog zu der amino-terminalen Region ist.

5. Verwendung nach einem der vorstehenden Ansprüche, worin das Peptid PTH(1-34), CFF(1-34), PTH(1-84), PTH(3-34), PTH(1-38), PTH(1-44), das des humanen Hypercalcämiefaktors (1-141) oder das des humanen Malignität-Hypercalcämiefaktors (1-40) ist.

6. Verwendung eines Peptids mit 10 % oder mehr Homologie zur Region der 34 amino-terminalen Aminosäuren des humanen Parathyroidhormons (PTH) oder zur Region der 34 amino-terminalen Aminosäuren des humanen Hypercalcämiefaktors, wobei das Peptid die Inhibierung der Vermehrung von kultivierten humanen Keratinocyten durch PTH(1-34), 1,25(OH)₂D₃ oder CFF(1-34) blockieren oder die Stimulierung der Differenzierung in vitro von kultivierten humanen Keratinocyten durch PTH(1-34), 1,25(OH)₂D₃ oder CFF(1-34) blockieren kann, zur Herstellung eines Mittels zur Induzierung der Vermehrung von Säugetierzellen.

7. Verwendung nach Anspruch 6 zur Herstellung eines Mittels zur Förderung des Hautwachstums bei einem Patienten mit Verbrennungen, Hautkrebserkrankungen oder Wunden, zur Stimulierung der Regenerierung der Epidermis bei einem Patienten mit verminderter Regenerationsfähigkeit aufgrund von Alter oder zur Stimulierung des Haarwachstums.

8. Verwendung nach Anspruch 6 oder 7, worin das Peptid zu mehr als 50 % zu der amino-terminalen Region homolog ist.

9. Verwendung nach einem der Ansprüche 6 bis 8, worin das Peptid PTH(7-34) ist.

## Revendications

1. Utilisation d'un peptide ayant 10% d'homologie, ou davantage, avec la région de 34 acides aminés à extrémité terminale amino de l'hormone parathyroïdienne humaine (PTH) ou avec la région de 34 acides aminés à extrémité terminale amino du facteur hypercalcémique humain, le peptide étant capable de rehausser la différenciation et d'inhiber la prolifération *in vitro* de kératinocytes humains cultivés, dans la préparation d'un agent pour inhiber la prolifération et rehausser la différenciation d'une cellule de mammifère.

2. Utilisation selon la revendication 1, dans la préparation d'un agent pour le traitement d'une maladie cutanée hyperproliférative ou pour le traitement du cancer.

3. Utilisation selon la revendication 2, dans la préparation d'un agent pour le traitement du psoriasis.

4. Utilisation selon l'une quelconque des revendications précédentes, dans laquelle le peptide a plus de 50% d'homologie avec ladite région à extrémité terminale amino.

5. Utilisation selon l'une des revendications précédentes, dans laquelle le peptide est la PTH(1-34), le CFF(1-34), la PTH(1-84), la PTH(3-34), la PTH(1-38), la PTH(1-44), le facteur hypercalcémique humain (1-141) ou le facteur humain d'hypercalcémie de malignité (1-40).

6. Utilisation d'un peptide ayant 10% d'homologie, ou davantage, avec la région de 34 acides aminés à extrémité terminale amino de l'hormone parathyroïdienne humaine (PTH) ou avec la région de 34 acides aminés à extrémité terminale amino du facteur hypercalcémique humain, le peptide étant capable de bloquer l'inhibition de la prolifération de kératinocytes humains cultivés par la PTH(1-34), la 1,25(OH)₂D₃ ou le CFF(1-34) ou de bloquer la stimulation de la différenciation *in vitro* de kératinocytes humains cultivés par la PTH(1-34), la 1,25(OH)₂D₃, ou le CFF(1-34), dans la préparation d'un agent pour induire la prolifération d'une cellule de mammifère.

7. Utilisation selon la revendication 6 dans la préparation d'un agent pour favoriser la croissance cutanée chez un patient présentant des brûlures, des ulcérations cutanées, ou des plaies, pour stimuler la recroissance épidermique chez un patient présentant une activité proliférative diminuée due au vieillissement, ou pour stimuler la pousse des cheveux.

8. Utilisation selon la revendication 6 ou la revendication 7, dans laquelle le peptide a plus de 50% d'homologie avec ladite région à extrémité terminale amino.

9. Utilisation selon l'une des revendications 6 à 8, dans laquelle le peptide est la PTH(7-34).
